# EUROPEAN PATENT SPECIFICATION

(11) **EP 0 439 431 B1**
(45) Date of publication and mention of the grant of the patent: **18.11.1993**
(21) Application number: 91810041.3
(22) Date of filing: 17.01.1991
(51) Int. Cl.: C07C 323/52, C10M 135/26

(54) **Thioalkanoic acid substituted N,N-dialkylhydroxylamines and stabilized lubricant compositions**
Carboxyalkylthio-substituierte N,N-Dialkylhydroxylamine und stabilisierte Schmiermittelzusammensetzungen
N,N-dialkylhydroxylamines substituées par le groupement carboxyalkylthio et compositions lubrifiantes stabilisées

(30) Priority: 26.01.1990 US 471167
(43) Date of publication of application: 31.07.1991
(73) Proprietor: CIBA-GEIGY AG, 4002 Basel (CH)
(72) Inventor: Evans, Samuel, Dr., CH-1723 Marly (CH); Chasan, David, Teaneck, N.J. 07666 (US); Seltzer, Raymond, Dr., New City, N.Y. 10956 (US)

(56) References cited:
- EP-A- 0 286 595
- EP-A- 0 349 380
- US-A- 4 612 393
- US-A- 4 720 517

## Description

This invention relates to lubricant compositions which are stabilized against oxidative decomposition by the addition of certain N,N-dialkylhydroxylamines which are substituted with thioalkanoic acid residues. This invention also relates to novel compounds.

### Background of the Invention

Various hydroxylamine derivatives have been disclosed as stabilizers for a variety of substrates. U.S. Pat. Nos. 4,720,517 4,612,393, 3,644,278 and 3,778,464 describe the use of substituted hydroxylamines of varying structures as antioxidant stabilizers for hydrocarbons including lubricating oils. U.S. Pat No. 3,408,422 discloses the use of selected hydroxylamine derivatives in unsaturated polyester compositions to prevent premature gelation on storage. U.S. Pat. No. 4,590,231 discloses the use of N,N-dibenzylhydroxylamine and other selected hydroxylamine derivatives in stabilizing polyolefin compositions. U.S. Pat. No. 4,242,224 discloses that the pink discoloration which occurs in the amine antioxidant and antiozonant emulsions used in the latex industry at high pH can be prevented or retarded by the use of dialkylhydroxylamines or mercaptan stabilizers. U.S. Pat. No. 4,316,996 pertains to a process of preparing phenolic antioxidants in the presence of a hydroxylamine derivative and of a substituted oxime to yield a phenolic antioxidant which itself exhibits improved color and color stability. U.S. Pat. No. 4,547,532 relates to the use of triorganotins and nitrogen compounds including hydroxylamines in the prevention of gelation of paint formulations. Neither the specific hydroxylamine derivatives disclosed herein nor the instantly disclosed lubricant compositions are disclosed in these patents.

Some bis(substituted thioalkyl)hydroxylamines are known. K. Ito et al., Chem. Pharm. Bull., 27, 1691 (1979) describes the Mannich type condensation of selected mercaptans, formaldehyde and hydroxylamine to yield compounds derived from propyl mercaptan, amyl mercaptan, cyclohexyl mercaptan, benzyl mercaptan, thiophenol and ethylene dimercaptan. Related compounds derived from N-methylhydroxylamine, N-benzylhydroxylamine and N-phenylhydroxylamine are also described. G. Rawson et al., Tetrahedron, 26, 5653 (1970) describe the preparation of N,N-(di-p-tolylthiomethyl)hydroxylamine and of N,N'-(di-p-bromophenylthiomethyl)-hydroxylamine in an academic study. No practical use for said compounds is disclosed. The possible use of such compounds as stabilizers for lubricants or other substrates is not suggested or described by the authors who were interested in the possible pharmaceutical applications of said compounds.

### Summary of the Invention

It has now been discovered that certain thioalkanoic acid substituted N,N-dialkylhydroxylamine compounds show surprisingly high stabilizer activity and sufficient solubility in lubricants. The COX moiety provided allows one to modify the compound so as to optimize its compatibility with various substrates.

Thus, the subject matter of the instant invention relates to compounds of formula I and to lubricant compositions comprising a mineral oil or a synthetic fluid or mixtures thereof and a stabilizing amount of a compound of formula I 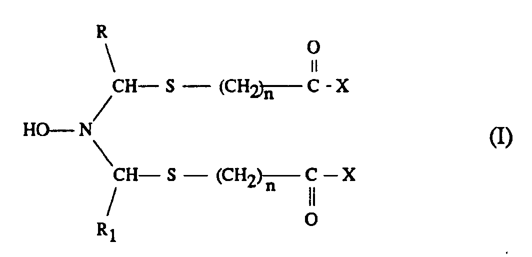
wherein R and R₁ are independently H, C₁-C₁₈-alkyl, or C₆-C₁₀-aryl, X is independently OR₂, SR₂ or NR₃R_{4,} R₂ is H, C₁-C₂₄-alkyl, C₅-C₁₂-cycloalkyl, C₇-C₉-aralkyl or -(CH₂CH₂O)ₘCH₂CH₂OR₅ wherein m is 0-6 and R₅ is C₁-C₁₈-alkyl, n is 1 or 2, and R₃ and R₄ are independently H, C₁-C₁₈-alkyl or C₆-C₁₀-aryl.

The various R groups may be C₁-C₁₈ straight-or branched-chain alkyl, *e.g*., methyl, ethyl, propyl, isopropyl, n-butyl, isobutyl, n-pentyl, isoamyl, n-hexyl, 2-ethylbutyl, n-octyl, 2-ethylhexyl, isononyl, n-decyl, isoundecyl, n-dodecyl, 2,4-dimethylpentyl, 2,4,6-trimethylheptyl, n-hexadecyl or n-octadecyl. As cycloalkyl, they may also be cyclopentyl or cyclohexyl; as aryl, they may be phenyl or naphthyl and as aralkyl they may be benzyl, α-methylbenzyl or α,α-dimethylbenzyl.

Examples of R₂ as polyethyleneoxy are -(CH₂CH₂O)₃CH₂CH₂OC₆H₁₃ and -(CH₂CH₂O)₃CH₂CH₂OC₁₂H₂₅. Examples of R₂ as NR₃R₄ are N(C₈H₁₇)₂ and N(C₆H₅)₂.

R and R₁ are preferably H. The index n is preferably 1 and R₂ is preferably C₈H₁₇, *e.g*. 2-ethylhexyl or iso-octyl, or R₂ is C₁₃H₂₇.

The compounds of formula I can be prepared by the Mannich reaction using an appropriately substituted mercaptocarboxylic acid, hydroxylamine hydrohalide, and an aldehyde. These raw materials are items of commerce or can be prepared by known methods.

The lubricant may be an oil or a grease based on mineral or synthetic fluids. These lubricants are well known to those skilled in the art. They are described, for example in D. Klamann, "Lubricants and Related Products", Verlag Chemie, Weinheim/Deerfield Beach, Basel 1984. The term mineral oil includes all mineral oils used for lubricant purposes, such as hydrocarbon mineral oils. The synthetic fluid may be, for example, an aliphatic or aromatic carboxylic ester or polymeric ester, a polyalkylene oxide, a phosphoric acid ester, polyalpha-olefins, or a silicone. Greases may be obtainable from these by adding metal soaps or other thickeners.

The amount of compound of formula I added to the lubricant depends on the sensitivity of the oil base to oxidation and on the desired degree of protection. Generally, 0.01 to 2% by weight based on the lubricant will be added, and preferably 0.05 to 0.5%.

The compounds of formula I may be used in combination with other antioxidants known as oil additives. Examples thereof are aromatic amines, hindered phenols, aliphatic or aromatic phosphates or phosphites, esters of thiodipropionic or thiodiacetic acid or salts of dithiocarbamic or dithiophosphoric acids. The lubricant composition may also contain other additives, such as metal-passivating agents, rust inhibitors, viscosity regulators, pour point depressants, anti-wear additives, dispersing agents or detergents, said additives being widely known and used in lubricants.

In the following list, representative additives are mentioned:

### Examples of phenolic antioxidants

1. Alkylated Monophenols
   2,6-Di-tert-butyl-4-methylphenol, 2,6-di-tert-butylphenol, 2-tert-butyl-4,6-dimethylphenol, 2,6-di-tert-butyl-4-ethylphenol, 2,6-di-tert-butyl-4-n-butylphenol, 2,6-di-tert-butyl-4-i-butylphenol, 2,6-di-cyclcopentyl-4-methylphenol, 2-(β-methylcyclohexyl)-4,6-dimethylphenol, 2,6-di-octadecyl-4-methylphenol, 2,4,6-tri-cyclohexylphenol, 2,6-di-tert-butyl-4-methoxymethylphenol, o-tert-butylphenol.
2. Alkylated Hydroquinones
   2,6-Di-tert-butyl-4-methoxyphenol, 2,5-di-tert-butyl-hydroquinone, 2,5-di-tert-amyl-hydroquinone, 2,6-diphenyl-4-octadecyloxyphenol.
3. Hydroxylated Thiodiphenylethers
   2,2'-Thio-bis-(6-tert-butyl-4-methylphenol), 2,2'-thio-bis-(4-octyl-phenol), 4,4'-thio-bis-(6-tert-butyl-3-methylphenol), 4,4'-thio-bis-(6-tert-butyl-2-methylphenol).
4. Alkylidene-Bisphenols
   2,2'-Methylene-bis-(6-tert-butyl-4-methylphenol), 2,2'-methylene-bis-(6-tert-butyl-4-ethylphenol), 2,2'-methylene-bis-(4-methyl-6-(α-methylcyclo- hexyl)-phenol), 2,2'-methylene-bis-(4-methyl-6-cyclohexylphenol), 2,2'-methylene-bis-(6-nonyl-4-methylphenol), 2,2'-methylene-bis-(4,6-di-tert-butylphenol), 2,2'-ethylidene-bis-(4,6-di-tert-butylphenol), 2,2'-ethylidene-bis-(6-tert-butyl-4- or -5-isobutylphenol), 2,2'-methylene-bis-(6-(α-methylbenzyl-4-nonylphenol), 2,2'-methylene-bis-(6-(α,α-dimethylbenzyl)-4-nonylphenol), 4,4'-methylene-bis-(2,6-di-tert-butylphenol), 4,4'-methylene-bis-(6-tert-butyl-2-methylphenol), 1,1-bis-(5-tert-butyl-4-hydroxy-2-methylphenol)-butane, 2,6-di-(3-tert-butyl-5-methyl-2-hydroxybenzyl)-4-methylphenol, 1,1,3-tris-(5-tert-butyl-4-hydroxy-2-methylphenyl)-3-n-dodecy l)-mercaptobutane, ethyleneglycol-bis-[3,3-bis-(3'-tert-butyl-4'-hydroxyphenyl)- butyrate], bis-(3-tert-butyl-4-hydroxy-5-methylphenyl)-dicyclopentadiene, bis-[2-(3'-tert-butyl-2'-hydroxy-5'-methyl-benzyl)-6-tert-butyl-4-methyl-phenyl]-terephthalate.
5. Benzyl Compounds
   1,3,5-Tri-(3,5-di-tert-butyl-4-hydroxybenzyl)-2,4,6-trimeth yl-benzene, bis-(3,5-di-tert-butyl-4-hydroxybenzyl)-sulfide, 3,5-di-tert-butyl-4-hydroxybenzyl-mercaptoacetic acid-isooctylester, bis-(4-tert-butyl-3-hydroxy-2,6-dimethylbenzyl)dithiolterepht halate, 1,3,5-tris-(3,5-di-tert-butyl-4-hydroxybenzyl)-isocyanurate, 1,3,5-tris-(4-tert-butyl-3-hydroxy-2,6-dimethylbenzyl)-isocyanurate, 3,5-di-tert-butyl-4-hydroxybenzyl-phosphonic acid-dioctadecylester, 3,5-di-tert-butyl-4-hydroxybenzyl-phosphonic acid-monoethylester, calcium-salt.
6. Acylaminophenols
   4-Hydroxy-lauric acid anilide, 4-hydroxy-stearic acid anilide, 2,4-bis-octylmercapto-6-(3,5-di-tert-butyl-4-hydroxyanilino)-s-triazine, N-(3,5-di-tert-butyl-4-hydroxyphenyl)-carbamic acid octyl ester.
7. Esters of β-(3,5-Di-tert-butyl-4-hydroxyphenol)-propionic acid with mono- or polyhydric alcohols, for example with methanol, diethyleneglycol, octadecanol, triethyleneglycol, 1,6-hexanediol, pentaerythritol, neopentylglycol, tris-hydroxyethyl-isocyanurate, thiodiethyleneglycol, bis-hydroxyethyl-oxalic acid diamide.
8. Esters of β-(5-tert-butyl-4-hydroxy-3-methylphenyl)-propionic acid with mono- or polyhydric alcohols, for example with methanol, diethyleneglycol, octadecanol, triethyleneglycol, 1,6-hexanediol, pentaerythritol, neopentylglycol, tris-hydroxyethyl-isocyanurate, thiodiethyleneglycol, di-hydroxyethyl-oxalic acid diamide.
9. Amides of β-(3,5-Di-tert-butyl-4-hydroxyphenyl)-propionic acid for example N,N'-Bis-(3,5-di-tert-butyl-4-hydroxyphenylpropionyl)-hexamet hylene-diamine, N,N'-bis-(3,5-di-tert-butyl-4-hydroxyphenylpropionyl)-tri-methylene-di amine, N,N'-bis-(3,5-di-tert-butyl-4-hydroxyphenylpropionyl)-hydrazi ne.

### Examples of amine antioxidants:

N,N'-Di-isopropyl-p-phenylenediamine, N,N'-di-sec.-butyl-p-phenylene-diamine, N,N'-bis(1,4-dimethyl-pentyl)-p-phenylenediamine, N,N'-bis(1-ethyl-3-methyl-pentyl)-p-phenylenediamine, N,N'-bis(1-methyl-heptyl)-p-phenylenediamine, N,N'-dicyclohexyl-p-phenylenediamine, N,N'-diphenyl-p-phenylenediamine, N,N'-di-(naphthyl-2-)-p-phenylenediamine, N-isopropyl-N'-phenyl-p-phenylenediamine, N-( 1,3-dimethyl-butyl)-N'-phenyl-p-phenylenediamine, N-(1-methyl-heptyl)-N'-phenyl-p-phenylenediamine, N-cyclohexyl-N'-phenyl-p-phenylenediamine, 4-(p-toluene-sulfonamido)-diphenylamine, N,N'-dimethyl-N,N'-di-sec-butyl-p-phenylenediamine, diphenylamine, N-allyldiphenylamine, 4-isopropoxy-diphenylamine, N-phenyl-1-naphthylamine, N-phenyl-2-naphthylamine, octylated diphenylamine, e.g. p,p'-di-tert-octyl-diphenylamine, 4-n-butylaminophenol, 4-butyrylamino-phenol, 4-nonanoylamino-phenol, 4-dodecanoylamino-phenol, 4-octadecanoyl-amino-phenol, di-(4-methoxy-phenyl)-amine, 2,6-di-tert-butyl-4-dimethyl-amino-methyl-phenol, 2,4'-diamino-diphenylmethane, 4,4'-diamino-diphenyl-methane, N,N,N',N'-tetramethyl-4,4'-diamino-diphenylmethane, 1,2-di- (phenylamino)-ethane, 1,2-di-[2-methyl-phenyl)-amino]-ethane, 1,3-di-(phenylamino)-propane, (o-tolyl)-biguanide, di-[4-( 1',3'-dimethyl-butyl)-phenyl]amine, tert-octylated N-phenyl-1-naphthylamine, mixture of mono- and dialkylated tert-butyl-/tert-octyldiphenylamines, 2,3-dihydro-3,3-dimethyl-4H-1,4-benzothiazine, phenothiazine, n-allylphenothiazine.

### Examples for other antioxidants:

Aliphatic or aromatic phosphites, esters of thiodipropionic acid or of thiodiacetic acid, or salts of dithiocarbamic or dithiophosphoric acid.

### Examples of metal deactivators, for example for copper, are:

Triazoles, benzotriazoles and derivatives thereof, tolutriazole and derivatives thereof, 2-mercaptobenzothiazole, 2-mercaptobenzotriazole, 2,5-dimercaptothiadiazole, 2,5-dimercaptobenzotriazole, 5,5'-methylene-bis-benzotriazole, 4,5,6,7-tetrahydrobenzotriazole, salicylidene-propylene-diamine and salicylaminoguanidine and salts thereof.

### Examples of rust inhibitors are:

a) Organic acids, their esters, metal salts and anhydrides, e.g. N-oleoyl-sarcosine, sorbitan-mono-oleate, lead-naphthenate, alkenyl-succinic acids and -anhydrides, e.g. dodecenyl-succinic acid anhydride, succinic acid partial esters and amides, 4-nonyl-phenoxy-acetic acid.
b) Nitrogen-containing compounds, e.g.
   I. Primary, secondary or tertiary aliphatic or cycloaliphatic amines and amine-salts of organic and inorganic acids, e.g. oil-soluble alkylammonium carboxylates
   II. Heterocyclic compounds, e.g. substituted imidazolines and oxazolines.
c) Phosphorus-containing compounds, e.g.
   Amine salts of phosphonic acid or phosphoric acid partial esters, zinc dialkyldithio phosphates.
d) Sulfur-containing compounds, e.g.
   Barium-dinonylnaphthalene-n-sulfonates, calcium petroleum sulfonates.

### Examples of viscosity-index improvers are:

Polyacrylates, polymethacrylates, vinylpyrrolidone/methacrylate-copolymers, polyvinylpyrrolidones, polybutenes, olefin-copolymers, styrene/acrylate-copolymers, polyethers.

### Examples of pour-point depressants are:

Polymethacrylates, alkylated naphthalene derivatives.

### Examples of dispersants/surfactants are:

Polybutenylsuccinic acid-amides or -imides, polybutenylphosphonic acid derivatives, basic magnesium-, calcium-, and bariumsulfonates and -phenolates.

### Examples of anti-wear additives are:

Sulfur- and/or phosphorus- and/or halogen-containing compounds eg sulfurised vegetable oils, zinc dialkyldithiophosphates, tritolylphosphate, chlorinated paraffins, alkyl- and aryldi- and trisulfides, triphenylphos- phorothionate, diethanolaminomethyltolutriazole, di(2-ethylhexyl)-aminomethyltolutriazole.

The following examples will further illustrate the embodiments of the instant invention.

### EXAMPLE 1

N,N-bis(2-ethylhexyloxycarbonylmethylthiomethyl) hydroxylamine

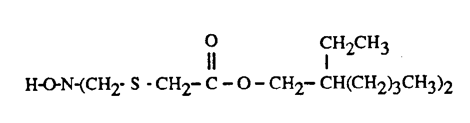

To a stirred suspension of 20.85 g of hydroxylamine hydrochloride in 1200 mL of methanol at 20°C is added a solution of 20. 8 g of potassium hydroxide in 300 mL of methanol. Aqueous formaldehyde solution (36%, 50 g) is added dropwise over a 15 minute period. Then 122. 6 g of mercaptoacetic acid 2-ethylhexyl ester are added over a further 15 minute period. The resulting white suspension is filtered and the filter cake is washed with methanol. The combined liquors are then evaporated to give a colorless oil. The oil is taken up in 500 mL of toluene and washed with two 300 mL portions of water. Evaporation of the solvent gives 132.2 g of liquid product.

Analysis: Calc'd. for C₂₂H₄₃NO₅S₂: C, 56.7; H, 9.3; N, 3.0; S, 13.8;

Found: C, 56.3; H, 9.4; N, 3.0; S, 13.8.

### EXAMPLE 2

N,N-bis(isooctyloxycarbonylmethylthiomethyl) hydroxylamine

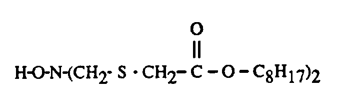

To a stirred suspension of 20.85 g of hydroxylamine hydrochloride in 1200 mL of methanol at 20°C is added a solution of 20.8 g of potassium hydroxide in 300 mL of methanol. Aqueous formaldehyde solution (36%, 50 g) is added dropwise over a 15 minute period. Then 122.6 g of thioglycolic acid 2-isooctyl ester (prepared from a mixture of C₈ alcohols) are added over a further 15 minute period. The resulting white suspension is filtered and the filter cake is washed with methanol. The combined liquors are then evaporated to give a colorless oil. The oil is taken up in 500 mL of toluene and washed with two 300 mL portions of water. Evaporation of the solvent gives 132.2 g of liquid product.

### EXAMPLE 3

Minimization of Coke Formation in Aircraft Turbine Oil

The following example illustrates the coke reducing activity of the instant stabilizers.

Stabilizers are blended into the base formulation at the indicated concentrations and tested according to the Federal Test Method No. 791B 3462. This method is used to determine the tendency of finished oils to form coke (solid decomposition products) when in contact with surfaces at elevated temperatures for relatively short periods. It consists of mechanically splashing the oil against a preheated plate under the following prescribed conditions:
Time 24 hr.
Panel Temperature 340 °C
Oil Splasher Shaft Speed 1,000 rpm
Sump Temperature 150 °C
Air Temperature 300 °C
Air Flow 1L/min.

Base Formulation Formulated, synthetic, 5 centiStoke., Aircraft Turbine Engine Lubricating Oil (ATO)

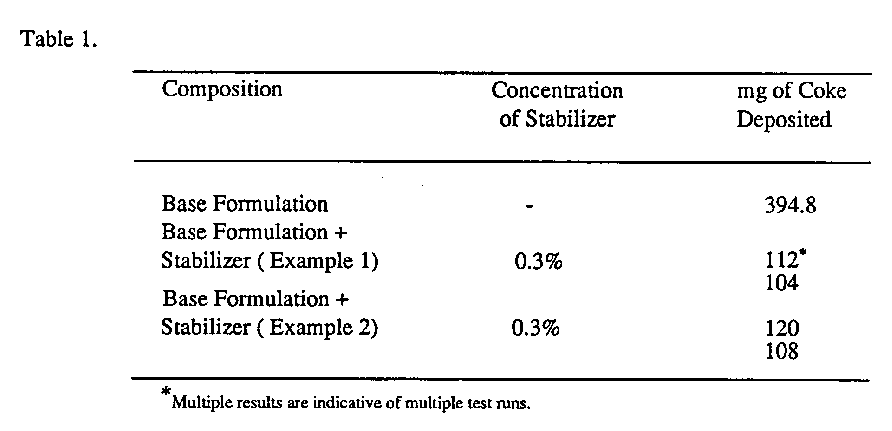

It is clear from the data above that small amounts of the instant stabilizer significantly reduce the level of coke formation in commercial jet turbine oil.

## Claims (Claims for the following Contracting State(s): DE, FR, GB, IT)

1. A compound of formula I 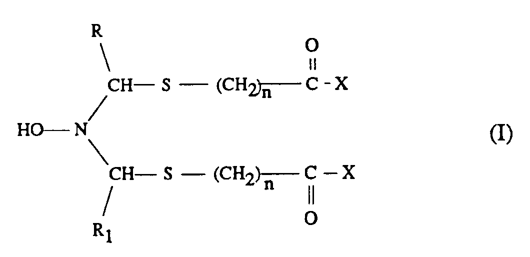 wherein R and R₁ are independently H, C₁-C₁₈-alkyl, or C₆-C₁₀-aryl, X is independently OR₂, SR₂ or NR₃R₄, R₂ is H, C₁-C₂₄-alkyl, C₅-C₁₂-cycloalkyl, C₁-C₉-aralkyl, or -(CH₂CH₂O)ₘCH₂CH₂OR₅ wherein m is 0-6 and R₅ is C₁-C₁₈alkyl, n is 1 or 2, and R₃ and R₄ are independently H, C₁-C₁₈-alkyl or C₆-C₁₀-aryl.

2. A compound according to claim 1 wherein R and R₁ are H.

3. A compound according to claim 2 wherein R₂ is C₈-alkyl when X is OR₂ or SR₂, and n is 1.

4. The compound according to claim 1 which is N,N-bis(2-ethylhexyloxycarbonylmethylthiomethyl)hydroxylamine.

5. The compound according to claim 1 which is N,N-bis(isooctyloxycarbonylmethylthiomethyl)hydroxylamine.

6. A lubricant composition comprising a mineral oil or a synthetic fluid or mixtures thereof and an effective stabilizing amount of a compound of formula (I) 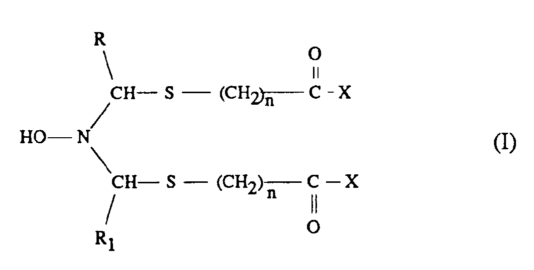 wherein R and R₁ are independently H, C₁-C₁₈-alkyl, or C₆-C₁₀-aryl, X is independently OR₂, SR₂ or NR₃R₄, R₂ is H, C₁-C₂₄-alkyl, C₅-C₁₂-cycloalkyl, C₇-C₉-aralkyl or -(CH₂CH₂O)ₘCH₂CH₂OR₅ wherein m is 0-6 and R₅ is C₁-C₁₈alkyl, n is 1 or 2, and R₃ and R₄ are independently H, C₁-C₁₈-alkyl or C₆-C₁₀-aryl.

7. A lubricant composition according to claim 6 containing a compound of formula I wherein R and R₁ are H.

8. A lubricant composition according to claim 7 containing a compound of formula I wherein R₂ is C₈-alkyl when X is OR₂ or SR₂, and n is 1.

9. A lubricant composition according to claim 6, wherein the compound of formula I is N,N-bis(2-ethylhexyloxycarbonylmethylthiomethyl)hydroxylamine.

10. A method of stabilizing lubricants against oxidative degradation which comprises adding thereto an effective stabilizing amount of a compound of formula I according to claim 1

## Claims (Claims for the following Contracting State(s): ES)

1. A method of stabilizing lubricants against oxidative degradation which comprises adding thereto a compound of formula I 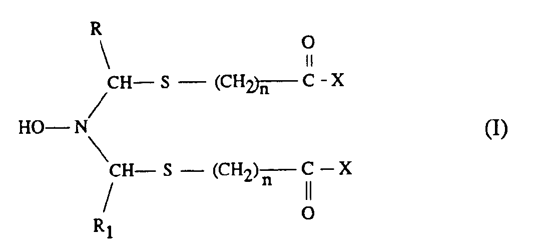 wherein R and R₁ are independently H, C₁-C₁₈-alkyl, or C₆-C₁₀-aryl, X is independently OR₂, SR₂ or NR₃R₄, R₂ is H, C₁-C₂₄-alkyl, C₅-C₁₂-cycloalkyl, C₇-C₉-aralkyl, or -(CH₂CH₂O)ₘCH₂CH₂OR₅ wherein m is 0-6 and R₅ is C₁-C₁₈alkyl, n is 1 or 2, and R₃ and R₄ are independently H, C₁-C₁₈-alkyl or C₆-C₁₀-aryl.

2. A method of stabilizing lubricants against oxidative degradation which comprises adding thereto a compound of formula I according to claim 1 wherein R and R₁ are H.

3. A method of stabilizing lubricants against oxidative degradation which comprises adding thereto a compound of formula I according to claim 2 wherein R₂ is C₈-alkyl when X is OR₂ or SR₂, and n is 1.

4. A method of stabilizing lubricants against oxidative degradation which comprises adding thereto a compound of formula I according to claim 1 which is N,N-bis (2-ethylhexyloxycarbonylmethylthiomethyl)hydroxylamine.

5. A method of stabilizing lubricants against oxidative degradation which comprises adding thereto a compound of formula I according to claim 1 which is N,N-bis(isooctyloxycarbonylmethylthiomethyl)hydroxylamine.

## Patentansprüche (Patentansprüche für folgende(n) Vertragsstaat(en): DE, FR, GB, IT)

1. Verbindung der Formel I 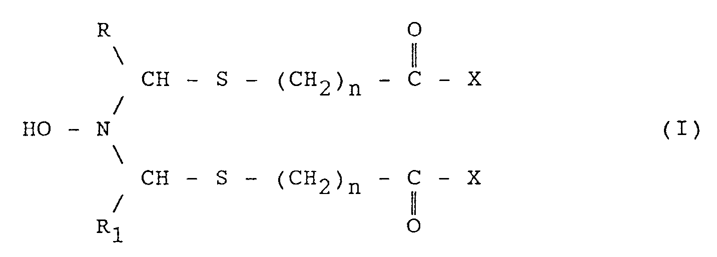 worin R und R₁ unabhängig voneinander ein Wasserstoffatom, C₁-C₁₈-Alkyl oder C₆-C₁₀-Aryl bedeuten, X unabhängig OR₂, SR₂ oder NR₃R₄ bedeutet, R₂ ein Wasserstoffatom, C₁C₂₄-Alkyl, C₅-C₁₂-Cycloalkyl, C₇-C₉-Aralkyl oder (CH₂CH₂O)ₘCH₂CH₂OR₅ darstellt, worin m 0-6 bedeutet und R₅ C₁-C₁₈-Alkyl darstellt, n 1 oder 2 ist und R₃ und R₄ unabhängig voneinander ein Wasserstoffatom, C₁-C1₈-Alkyl oder C₆-C₁₀-Aryl bedeuten.

2. Verbindung nach Anspruch 1, worin R und R₁ Wasserstoffatome darstellen.

3. Verbindung nach Anspruch 2, worin R₂ C₈-Alkyl bedeutet, wenn X OR₂ oder SR₂ darstellt und n 1 ist.

4. Verbindung nach Anspruch 1, nämlich N,N-Bis(2-ethyl-hexyloxycarbonylmethylthiomethyl)hydroxylamin.

5. Verbindung nach Anspruch 1, nämlich N,N-Bis(isooctyloxycarbonylmethylthiomethyl)hydroxylamin.

6. Schmiermittelzusammensetzung, umfassend ein Mineralöl oder ein synthetisches Fluid oder Gemische davon und eine wirksam stabilisierende Menge einer Verbindung der Formel (I) 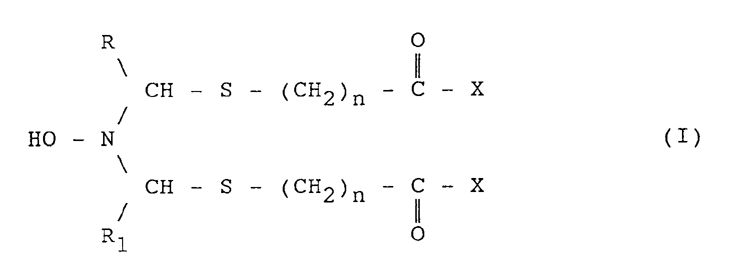 worin R und R₁ unabhängig voneinander ein Wasserstoffatom, C₁-C₁₈-Alkyl oder C₆-C₁₀-Aryl bedeuten, X unabhängig OR₂, SR₂ oder NR₃R₄ bedeutet, R₂ ein Wasserstoffatom, C₁-C₂₄-Alkyl, C₅-C₁₂-Cycloalkyl, C₇-C₉-Aralkyl oder (CH₂CH₂O)ₘCH₂CH₂OR₅ darstellt, worin m 0-6 bedeutet und R₅ C₁-C₁₈-Alkyl darstellt, n 1 oder 2 ist und R₃ und R₄ unabhängig voneinander ein Wasserstoffatom, C₁-C₁₈-Alkyl oder C₆-C₁₀-Aryl bedeuten.

7. Schmiermittelzusammensetzung nach Anspruch 6, enthaltend eine Verbindung der Formel I, worin R und R₁ Wasserstoffatome bedeuten.

8. Schmiermittelzusammensetzung nach Anspruch 7, enthaltend eine Verbindung der Formel I, worin R₂ C₈-Alkyl bedeutet, wenn X OR₂ oder SR₂ darstellt, und n 1 ist.

9. Schmiermittelzusammensetzung nach Anspruch 6, wobei die Verbindung der Formel I N,N-Bis(2-ethylhexyloxycarbonylmethylthiomethyl)hydroxylamin ist.

10. Verfahren zum Stabilisieren von Schmiermitteln gegen oxidativen Abbau, umfassend die Zugabe einer wirksam stabilisierenden Menge einer Verbindung der Formel I gemäß Anspruch 1.

## Patentansprüche (Patentansprüche für folgende(n) Vertragsstaat(en): ES)

1. Verfahren zum Stabilisieren von Schmiermitteln gegen oxidativen Abbau, umfassend die Zugabe einer Verbindung der Formel I 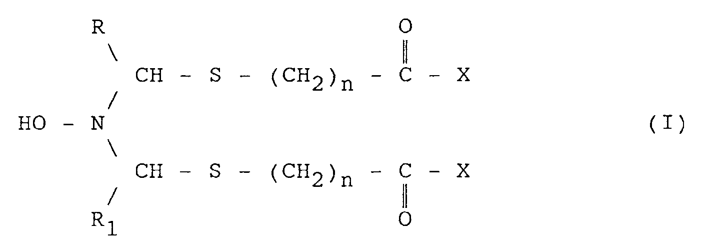 worin R und R₁ unabhängig voneinander ein Wasserstoffatom, C₁-C₁₈-Alkyl oder C₆-C₁₀-Aryl bedeuten, X unabhängig OR₂, SR₂ oder NR₃R₄ bedeutet, R₂ ein Wasserstoffatom, C₁-C₂₄-Alkyl, C₅-C₁₂-Cycloalkyl, C₇-C₉-Aralkyl oder -(CH₂CH₂O)ₘCH₂CH₂OR₅ darstellt, worin m 0-6 bedeutet und R₅ C₁-C₁₈-Alkyl darstellt, n 1 oder 2 ist und R₃ und R₄ unabhängig voneinander ein Wasserstoffatom, C₁-C₁₈-Alkyl oder C₆-C₁₀-Aryl bedeuten.

2. Verfahren zum Stabilisieren von Schmiermitteln gegen oxidativen Abbau, umfassend die Zugabe einer Verbindung der Formel I nach Anspruch 1, worin R und R₁ Wasserstoffatome darstellen.

3. Verfahren zum Stabilisieren von Schmiermitteln gegen oxidativen Abbau, umfassend die Zugabe einer Verbindung der Formel I nach Anspruch 2, worin R₂ C₈-Alkyl bedeutet, wenn X OR₂ oder SR₂ darstellt, und n 1 ist.

4. Verfahren zum Stabilisieren von Schmiermitteln gegen oxidativen Abbau, umfassend die Zugabe einer Verbindung der Formel I nach Anspruch 1, nämlich N,N-Bis(2-ethylhexyloxycarbonylmethylthiomethyl)hydroxylamin.

5. Verfahren zum Stabilisieren von Schmiermitteln gegenoxidativen Abbau, umfassend die Zugabe einer Verbindung der Formel I nach Anspruch 1, nämlich N,N-Bis(isooctyloxycarbonylmethylthiomethyl)hydroxylamin.

## Revendications (Revendications pour l'(les) Etat(s) contractant(s) suivant(s): DE, FR, GB, IT)

1. Composé répondant à la formule I. 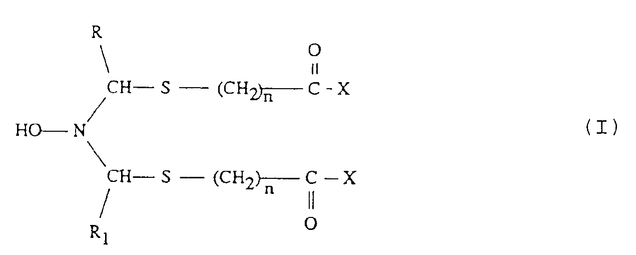 dans laquelle R et R₁ sont indépendamment H, un groupe alkyle en C₁-C₁₈ ou aryle en C₆-C₁₀, X est indépendamment OR₂, SR₂ ou NR₃R₄, R₂ est H, un groupe alkyle en C₁-C₂₄, cycloalkyle en C₅-C₁₂, aralkyle en C₇-C₉ ou -(CH₂CH₂O)ₘCH₂CH₂OR₅ dans laquelle m est un nombre de O à 6 et R₅ est un groupe alkyle en C₁-C₁₈, n est 1 ou 2 et R₃ et R₄ sont indépendamment H, un groupe alkyle en C₁-C₁₈ ou aryle en C₆-C₁₀.

2. Composé selon la revendication 1, dans lequel R et R₁ sont H.

3. Composé selon la revendication 2, dans lequel R₂ est un groupe alkyle en C₈ lorsque X est OR₂ ou SR₂ et n est 1.

4. Composé selon la revendication 1, qui est la N,N-bis(2-éthylhexyloxycarbonylméthylthiométhyl)-hydroxylamine.

5. Composé selon la revendication 1, qui est la N,N-bis(isooctyloxycarbonylméthylthiométhyl)-hydroxylamine.

6. Composition de lubrifiant comprenant une huile minérale ou un fluide synthétique ou des mélanges de ceux-ci et une quantité efficace comme stabilisant d'un composé répondant à la formule I : 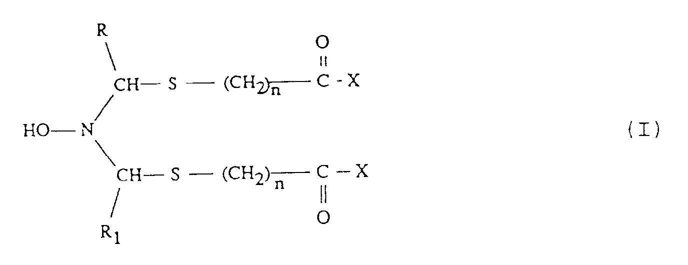 dans laquelle R et R₁ sont indépendamment H, un groupe alkyle en C₁-C₁₈ ou aryle en C₆-C₁₀, X est indépendamment OR₂, SR₂ ou NR₃R₄, R₂ est H, un groupe alkyle en C₁-C₂₄, cycloalkyle en C₅-C₁₂, aralkyle en C₇-C₉ ou -(CH₂CH₂O)ₘCH₂CH₂OR₅ dans laquelle m est un nombre de 0 à 6 et R₅ est un groupe alkyle en C₁-C₁₈, n est 1 ou 2 et R₃ et R₄ sont indépendamment H, un groupe alkyle en C₁-C₁₈ ou aryle en C₆-C₁₀.

7. Composition de lubrifiant selon la revendication 6 contenant un composé répondant à la formule I dans laquelle R et R₁ sont H.

8. Composition de lubrifiant selon la revendication 7 contenant un composé répondant à la formule I dans laquelle R₂ est un groupe alkyle en C₈ lorsque x est OR₂ ou SR₂ et n est 1.

9. Composition de lubrifiant selon la revendication 6 dans laquelle le composé répondant à la formule I est la N,N-bis(2-éthylhexyloxycarbonylméthylthiométhyl)-hydroxylamine.

10. Procédé de stabilisation de lubrifiants vis-à-vis de la dégradation oxydante, qui comprend l'addition à ceux-ci d'une quantité stabilisante efficace d'un composé répondant à la formule I selon la revendication 1.

## Revendications (Revendications pour l'(les) Etat(s) contractant(s) suivant(s): ES)

1. Procédé de stabilisation de lubrifiants vis-à-vis de la dégradation oxydante, qui comprend l'addition à ceux-ci d'un composé répondant à la formule I. 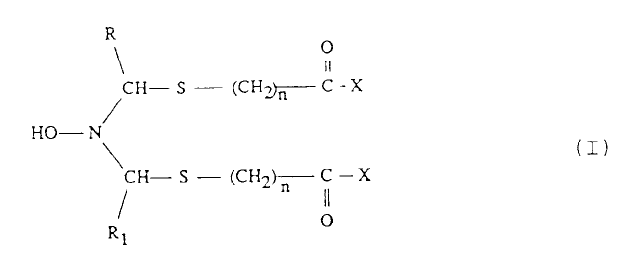 dans laquelle R et R₁ sont indépendamment H, des groupes alkyle en C₁-C₁₈ ou aryle en C₆-C₁₀, X est indépendamment OR₂, SR₂ ou NR₃R₄, R₂ est H, un groupe alkyle en C₁-C₂₄, cycloalkyle en C₅-C₁₂, aralkyle en C₇-C₉ ou -(CH₂CH₂O)ₘCH₂CH₂OR₅ dans laquelle m est un nombre de 0 à 6 et R₅ est un groupe alkyle en C₁-C₁₈, n est 1 ou 2 et R₃ et R₄ sont indépendamment H, un groupe alkyle en C₁-C₁₈ ou aryle en C₆-C₁₀.

2. Procédé de stabilisation de lubrifiants vis-à-vis de la dégradation oxydante, qui comprend l'addition à ceux-ci d'un composé répondant à la formule I suivant la revendication 1, dans laquelle R et R₁ sont H.

3. Procédé de stabilisation de lubrifiants vis-à-vis de la dégradation oxydante, qui comprend l'addition à ceux-ci d'un composé répondant à la formule I suivant la revendication 2, dans laquelle R₂ est un groupe alkyle en C₈ lorsque X est OR₂ ou SR₂, et n est 1.

4. Procédé de stabilisation de lubrifiants vis-à-vis de la dégradation oxydante, qui comprend l'addition à ceux-ci d'un composé répondant à la formule I suivant la revendication 1, qui est la N,N-bis(2-éthylhexyloxy- carbonylméthylthiométhyl)-hydroxylamine.

5. Procédé de stabilisation de lubrifiants vis-à-vis de la dégradation oxydante qui comprend l'addition à ceux-ci d'un composé répondant à la formule I selon la revendication 1, qui est la N,N-bis(isooctyloxycarbonylméthylthiométhyl)-hydroxylamine.
